# FASCICULE DE BREVET EUROPEEN

(11) **EP 0 973 802 B1**
(45) Date de publication et mention de la délivrance du brevet: **25.10.2006**
(21) Numéro de dépôt: 98920571.1
(22) Date de dépôt: 06.04.1998
(51) Int. Cl.: C07K 14/16, C12N 7/00

(54) **PEPTIDES SYNTHETIQUES UTILISABLES DANS LES ESSAIS BIOLOGIQUES POUR LA DETECTION DES INFECTIONS DUES AUX VIRUS VIH-1 DU GROUPE O**
SYNTHETISCHE PEPTIDE FÜR TESTVERFAHREN ZUR BESTIMMUNG DER INFEKTION MIT VIRUS HIV1 DER GRUPPE O
SYNTHETIC PEPTIDES USEFUL IN BIOLOGICAL ASSAYS FOR DETECTING INFECTIONS CAUSED BY GROUP O HIV-1 VIRUSES

(30) Priorité: 09.04.1997 FR 9704356; 24.02.1998 FR 9802212
(43) Date de publication de la demande: 26.01.2000
(73) Titulaire: Bio-Rad Pasteur, 92430 Marnes-la-Coquette (FR)
(72) Inventeur: CHENEBAUX, Denis, Marie, Bernard, F-78000 Versailles (FR); DELAGNEAU, Jean-François, Hubert, F-78170 La Celle Saint Cloud (FR); GADELLE, Stéphane, Jean, Xavier, F-91570 Bièvres (FR); RIEUNIER, François, Yves, F-78330 Fontenay le Fleury (FR)
(74) Mandataire: Bernasconi, Jean Raymond
(86) Numéro de dépôt international: PCT/FR1998/000691
(87) Numéro de publication internationale: WO 1998/045323

(56) Documents cités:
- WO-A-95/32293
- WO-A-96/12809
- WO-A-96/27012
- WO-A-96/27013

### LISTE DE SEQUENCES

(1) INFORMATION GENERALE:
   (i) DEPOSANT:
      (A) NOM: Pasteur Sanofi Diagnostics
      (B) RUE: 3 boulevard Raymond Poincaré
      (C) VILLE: Marnes la Coquette
      (E) PAYS: France
      (F) CODE POSTAL: 92430
      (G) TELEPHONE: 0153774000
      (H) TELECOPIE: 0153774133
   (ii) TITRE DE L' INVENTION: peptides synthétiques utilisables dans les essais biologiques pour la détection des infections dues au virus VIH-1 du groupe O
   (iii) NOMBRE DE SEQUENCES: 16
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 1:
   Leu Leu Ser Leu Trp Gly Cys Arg Gly Lys Ala Val Cys Tyr Thr Ser
   1               5                   10                  15
   Val Gln Trp Asn Glu Thr
               20
(2) INFORMATION POUR LA SEQ ID NO: 2:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 2:
   Leu Leu Ser Leu Trp Gly Cys Arg Gly Arg Leu Val Cys Tyr Thr Ser
   1               5                   10                  15
   Val Gln Trp Asn Glu Thr
               20
(2) INFORMATION POUR LA SEQ ID NO: 3:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 3:
   Leu Leu Ser Ser Trp Gly Cys Lys Gly Arg Leu Val Cys Tyr Thr Ser
   1               5                   10                  15
   Val Gln Trp Asn Glu Thr
               20
(2) INFORMATION POUR LA SEQ ID NO: 4:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 4:
   Leu Leu Ser Ser Trp Gly Cys Lys Gly Arg Leu Val Cys Tyr Thr Ser
   1               5                   10                  15
   Val Gln Trp Asn Ser Thr
               20
(2) INFORMATION POUR LA SEQ ID NO: 5:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 5:
   Leu Leu Gln Ser Trp Gly Cys Lys Gly Arg Leu Val Cys Tyr Thr Ser
   1               5                   10                  15
   Val Gln Trp Asn Ser Thr
               20
(2) INFORMATION POUR LA SEQ ID NO: 6:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 6:
   Leu Leu Asn Ser Trp Gly Cys Arg Gly Lys Ala Val Cys Tyr Thr Ser
   1               5                   10                  15
   Val Gln Trp Asn Glu Thr
               20
(2) INFORMATION POUR LA SEQ ID NO: 7:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 7:
   Leu Leu Ser Leu Trp Gly Cys Arg Gly Arg Ala Val Cys Tyr Thr Ser
   1               5                   10                  15
   Val Gln Trp Asn Glu Thr
               20
(2) INFORMATION POUR LA SEQ ID NO: 8:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 8:
   Leu Leu Ser Ser Trp Gly Cys Arg Gly Arg Leu Val Cys Tyr Thr Ser
   1               5                   10                  15
   Val Gln Trp Asn Glu Thr
               20
(2) INFORMATION POUR LA SEQ ID NO: 9:
   (i) CARACTERISTIQUES DE LA SEQUENCE:
      (A) LONGUEUR: 16 acides aminés
      (B) TYPE: acide aminé
   (ii) TYPE DE MOLECULE: peptide
   (xi) DESCRIPTION DE LA SEQUENCE: SEQ ID NO: 9:
   Leu Leu Ser Ser Trp Gly Cys Lys Gly Arg Leu Val Cys Tyr Thr Ser
   1               5                   10                  15
(2) INFORMATION POUR LA SEQ ID NO: 10:
   (i) CARACTERISTIQUES DE LA SEQUENCE:
      (A) LONGUEUR: 16 acides aminés
      (B) TYPE: acide aminé
   (ii) TYPE DE MOLECULE: peptide
   (xi) DESCRIPTION DE LA SEQUENCE: SEQ ID NO: 10:
   Leu Leu Asn Ser Trp Gly Cys Lys Gly Arg Leu Val Cys Tyr Thr Ser
   1 5 10 15
(2) INFORMATION POUR LA SEQ ID NO: 11:
   (i) CARACTERISTIQUES DE LA SEQUENCE:
      (A) LONGUEUR: 32 acides aminés
      (B) TYPE: acide aminé
   (ii) TYPE DE MOLECULE: peptide
   (xi) DESCRIPTION DE LA SEQUENCE: SEQ ID NO: 11:
   Ala Leu Glu Thr Leu Leu Gln Asn Gln Gln Leu Leu Asn Ser Trp Gly
   1 5 10 15
   Cys Arg Gly Arg Leu Val Cys Tyr Thr Ser Val Arg Trp Asn Glu Thr
   20 25 30
(2) INFORMATION POUR LA SEQ ID NO: 12:
   (i) CARACTERISTIQUES DE LA SEQUENCE:
      (A) LONGUEUR: 32 acides aminés
      (B) TYPE: acide aminé
   (ii) TYPE DE MOLECULE: peptide
   (xi) DESCRIPTION DE LA SEQUENCE: SEQ ID NO: 12:
   Ala Leu Glu Thr Leu Leu Gln Asn Gln Gln Leu Leu Asn Ile Trp Gly
   1 5 10 15
   Cys Arg Gly Arg Leu Val Cys Tyr Thr Ser Val Arg Trp Asn Glu Thr
   20 25 30
(2) INFORMATION POUR LA SEQ ID NO: 13:
   (i) CARACTERISTIQUES DE LA SEQUENCE:
      (A) LONGUEUR: 32 acides aminés
      (B) TYPE: acide aminé
   (ii) TYPE DE MOLECULE: peptide
   (xi) DESCRIPTION DE LA SEQUENCE: SEQ ID NO: 13:
   Ala Leu Glu Thr Leu Leu Gln Asn Gln Gln Leu Leu Asp Leu Trp Gly
   1 5 10 15
   Cys Arg Gly Arg Leu Val Cys Tyr Thr Ser Val Arg Trp Asn Glu Thr
   20 25 30
(2) INFORMATION POUR LA SEQ ID NO: 14:
   (i) CARACTERISTIQUES DE LA SEQUENCE:
      (A) LONGUEUR: 22 acides aminés
      (B) TYPE: acide aminé
   (ii) TYPE DE MOLECULE: peptide
   (xi) DESCRIPTION DE LA SEQUENCE: SEQ ID NO: 14:
   Leu Asn Gln Gln Arg Leu Leu Asn Ser Trp Gly Cys Lys Gly Arg Leu
   1 5 10 15
   Val Cys Tyr Thr Ser Val
   20
(2) INFORMATION POUR LA SEQ ID NO: 15 :
   (i) CARACTERISTIQUES DE LA SEQUENCE:
      (A) LONGUEUR: 28 acides aminés
      (B) TYPE: acide aminé
   (ii) TYPE DE MOLECULE: peptide
   (xi) DESCRIPTION DE LA SEQUENCE: SEQ ID-NO: 15 :
   Arg Ala Leu Glu Thr Leu Leu Asn Gln Gln Arg Leu Leu Asn Ser Trp
   1 5 10 15
   Gly Cys Lys Gly Arg Leu Val Cys Tyr Thr Ser Val
   20 25
(2) INFORMATION POUR LA SEQ ID NO: 16 :
   (i) CARACTERISTIQUES DE LA SEQUENCE:
      (A) LONGUEUR: 17 acides aminés
      (B) TYPE: acide aminé
   (ii) TYPE DE MOLECULE: peptide
   (xi) DESCRIPTION DE LA SEQUENCE: SEQ ID NO: 16:
   Arg Leu Asn Ser Trp Gly Cys Lys Gly Arg Leu Val Cys Tyr Thr Ser
   1               5                   10                  15
   Val

## Description

L'invention concerne des peptides synthétiques utilisables dans les essais biologiques pour la détection des infections dues aux virus VIH-1 du groupe O, leur procédé de préparation, des compositions et des trousses contenant de tels peptides ainsi que les essais biologiques mettant en oeuvre de tels peptides.

Des rétrovirus VIH-1 du groupe O sont connus dans l'art antérieur. Le brevet EP 0 345 375 et la demande de brevet EP 0 657 532 décrivent les isolats ANT 70 et ANT 70 NA isolés chez des malades Camerounais. Ces documents décrivent plus précisément des antigènes et des compositions antigéniques contenant des lysats ou des protéines de ces isolats, les acides nucléiques correspondant à l'ARN génomique, des méthodes d'hybridation mettant en oeuvre ces acides nucléiques, des méthodes de production des isolats dénommés ci-dessus ainsi que des procédés de préparation de protéines p12, p16, p25, gp41, gp120 de ces rétrovirus.

La demande EP 0 591 914 décrit l'isolat MVP 5180/91. Cet isolat caractérisé par Western Blot présente, comme l'isolat précédent, des différences par rapport aux isolats du rétrovirus VIH-1 connus depuis longtemps. La demande EP 0 591 914 décrit précisément la séquence d'ADN de l'isolat MVP 5180/91 et indique précisément la localisation des gènes gag, pol et env. La demande EP 0 591 914 décrit encore des peptides synthétiques de la boucle V3 ainsi que de la région immunodominante (gp41). Ces derniers sont utiles pour des essais biologiques, notamment pour la détection, *in vitro,* des anticorps VIH-1 du groupe O.

La demande EP 0 673 948 décrit des peptides synthétiques ou recombinés constitués de 15 à 50 acides aminés (AA) et comportant la séquence
-VWGIRQLRARLOALETLIONOORLNLWGXKGKLIXYTSVKWNTSWSGR- dans laquelle X représente soit un résidu de cystéine, soit un résidu de sérine. Ces peptides sont utiles dans le domaine diagnostique pour la détection des infections dues à certains isolats de rétrovirus VIH-1 du groupe O.

On connait également la demande EP 0 727 483 qui décrit l'isolat MVP 2901194 qui fait aussi partie des rétrovirus appartenant à la famille VIH-1 du groupe O. Cette demande décrit certains antigènes ayant des séquences peptidiques bien déterminées. Ces séquences peptidiques correspondent à une partie de la séquence de la gp120 et une partie de la gp41 (région immunodominante) de l'isolat MVP 2901/94.

La demande WO 96/12809 décrit deux nouveaux isolats appartenant à la famille VIH-1 du groupe O. Il s'agit des isolats VAU et DUR. Cette demande décrit certaines séquences peptidiques issues des deux virus cités ci-dessus, utiles pour la détection d'anticorps reconnaissant les séquences peptidiques VIH-1 VAU ou DUR.

La demande WO 96/32293 décrit deux antigènes issus de la séquence de l'isolat ANT 70. Il s'agit de l'antigène appelé MDL061 et de l'antigène MDL056, de la région immunodominante de la gp41. Selon cette invention, pour détecter 100% des échantillons d'une collection limitée de sérums de malades infectés par le virus de VIH-1 du groupe O, il est nécessaire d'utiliser des compositions contenant ces deux peptides, puisque chaque peptide isolé ne permet pas à lui seul d'obtenir des résultats satisfaisants.

En effet, il est quasi impossible, au regard de la variabilité génétique révélée par les isolats du virus du groupe O, de garantir le dépistage sérologique des individus contaminés par la mise en oeuvre d'antigènes issus du même et unique isolat. Cela signifie qu'il n'est pas possible d'obtenir des réactifs qui garantissent 100% de sensibilité. Le groupe O ainsi soulève pour la première fois un problème important ; il s'agit de l'inadéquation de certains réactifs sérologiques à reconnaître des individus contaminés par des groupes ou sous-types particulièrement divergents. C'est le cas justement des VIH1 du groupe O.

La demande WO 96/40763 insiste aussi sur la grande divergence du groupe O. Cette demande décrit des peptides qui incorporent, dans une séquence naturelle VIH-1 de type B, quelques petites modifications (remplacement d'un ou de deux acides aminés). Selon cette demande, ces peptides hybrides sont capables de réagir avec des anticorps anti groupe O.

La demande WO 96/27013 décrit une série de nouveaux virus VIH1 du groupe O désignés BCF 01, BCF 02, BCF 03, BCF06, BCF 07, BCF 08, BCF09, BCF11, BCF12, BCF13 et BCF14 ainsi qu'une série de peptides de la région dominante de la gp41 correspondante dénommés ESS/BCF02, FAN/BCF01, LOB/BCF06, MAN/BCF07, NKO/BCF08, POC/BCF03, NAN/BCF11, BCF09, BCF12, BCF13 et BCF14. Un certain nombre de ces peptides sont peu maniables en diagnostic à cause de leur faible solubilité, notamment le peptide BCF13.

De manière inattendue, il a été maintenant trouvé que certains peptides synthétiques sont des réactifs diagnostiques de qualité supérieure et permettent de dépister de manière satisfaisante les malades infectés par les rétrovirus VIH-1 du groupe O. Ces peptides sont composés de séquences variables articulées autour de courtes séquences très conservées, présentes dans les isolats des rétrovirus VIH-1 du groupe O. Les peptides de l'invention permettent d'obtenir des résultats bien supérieurs à ceux obtenus avec des peptides synthétiques porteurs d'épitopes immunodominants de la gp41 (env) de certains isolats VIH-1 du groupe O.

Par la suite, pour dénommer les acides aminés, on utilisera la nomenclature à trois lettres.

Les peptides synthétiques de l'invention sont de type monomère de 13 à 33 acides aminés ou de type dimère de 26 à 66 acides aminés, sous forme linéaire ou sous forme cyclisée par l'intermédiaire de ponts disulfures inter-cystéines, et répondent à la formule générale (I) :

Δ-Z-TrpGlyCys-Θ-CysTyrThrSer-Ω (I)

dans laquelle :
- Δ représente un radical biotinyle, un radical biocytinyle, un atome d'hydrogène, un radical acétyle (CH₃CO-), une chaîne aliphatique pouvant contenir une ou deux fonctions thiol, aldéhyde ou amine, la chaîne aliphatique étant de préférence une chaîne alkyle de 1 à 6 atomes de carbone ou une chaîne alcényle de 2 à 6 atomes de carbone, ou une chaîne aminoalkylcarbonyle de 2 à 6 atomes de carbone,
- Z représente une séquence peptidique d'une des formules (II) à (X) :

   -Ξ₁-Ser-Ξ₂- (II)

   -Ser-Ξ₂- (III)

   -Ξ₁-Ser- (IV)

   -Ξ₁-Gln-Ξ₂ (V)

   -Gln-Ξ₂- (VI)

   -Ξ₁-Gln- (VII)

   -Ξ₁-Asn-Ξ₂- (VIII)

   -Asn-Ξ₂- (IX)

   Ξ₁-Asn- (X)

   dans lesquelles :
   - Ξ₁, représente une séquence peptidique de 0 à 9 acides aminés et
   - Ξ₂ représente une séquence peptidique de 0 à 5 acides aminés,
- Θ représente une séquence peptidique de formule (XI) :

   -(AA₁)-(AA₂)-(AA₃)-(AA₄)-(AA₅)- (XI)

   dans laquelle :
   - (AA₁) représente soit un résidu lysine, soit un résidu arginine, soit un résidu ornithine,
   - (AA₂) représente soit un résidu glycine, soit un résidu asparagine,
   - (AA₃) représente soit un résidu lysine, soit un résidu arginine, soit un résidu ornithine,
   - (AA₄) représente soit un résidu leucine, soit un résidu isoleucine, soit un résidu glutamine,
   - (AA₅) représente soit un résidu isoleucine, soit un résidu valine, soit un résidu leucine, soit un résidu thréonine, soit un résidu norleucine, soit un résidu norvaline,
   à condition toutefois que (AA₁), (AA₂), (AA₃), (AA₄) et (AA₅) ne forment jamais ensemble les séquences peptidiques -Lys Gly Lys Leu Ile- et -Lys Gly Lys Leu Val-,
- Ω, fixé sur le groupe -CO- de la sérine représente :
   - un radical hydroxyle (-OH) ou un radical amino (-NH₂),
   - un radical alcoxy comportant de 1 à 6 atomes de carbone,
   - une séquence peptidique de formule (XII) :

      -Vat-Σ-Ψ (XII)

      dans laquelle Σ représente une séquence de formule (XIII) ou de formule (XIV) :

      -(AA₆)-Trp Asn-(AA₇)-(AA₈) (XIII)

      -(AA₆)-Trp His-(AA₇)-(AA₈) (XIV)

      dans lesquelles :
      - (AA₆) représente un acide aminé différent de la lysine,
      - (AA₇) représente un acide aminé,
      - (AA₈) représente un résidu sérine ou thréonine,
      et Ψ fixé sur le reste -CO- de l'acide aminé AA₈ libre, représente un groupe OH, NH₂ ou un radical alcoxy comportant de 1 à 6 atomes de carbone,
   - une séquence peptidique de formule (XV) :

      -Val-Ψ (XV)

      dans laquelle Ψ fixé sur le reste -CO- de la valine, a la même signification que pour la formule (XII),
   - ou une séquence peptidique d'une des formules (XVI) à (XVIII) :

      -Z-TrpGlyCys-Θ-CysTyrThrSer-Ψ (XVI)

      Val-Σ-Z-TrpGlyCys-Θ-CysTyrThrSerVal-Σ-Ψ (XVII)

      Val-Z-TrpGlyCys-Θ-CysTyrThrSerVal-Ψ (XVIII)
   dans lesquelles Z et Θ ont la définition donnée pour la formule (I) et Σ a la définition donnée pour la formule (XII) et Ψ fixé sur le reste -CO- de la sérine, sur le reste -CO- de l'acide aminé AA₈ ou sur le reste -CO- de la valine, a la même signification que pour la formule (XII).

On préfère les composés de formule (I) dans laquelle (AA₅) représente soit un résidu valine, soit un résidu leucine, soit un résidu thréonine, et lorsque Ω correspond à une séquence peptidique de formule (XII), (AA₆) représente soit un résidu glutamine, soit un résidu arginine.

On préfère les peptides de formule (I) dans laquelle:
- Δ représente un radical biotinyle, un atome d'hydrogène, ou une chaîne aliphatique pouvant contenir une ou deux fonctions thiol, aldéhyde ou amine, la chaîne aliphatique étant de préférence une chaîne alkyle de 1 à 6 atomes de carbone, ou une chaîne aminoalkylcarbonyle de 2 à 6 atomes de carbone,
- Z représente une séquence peptidique de formule (II) ou (V), dans lesquelles Ξ₁ représente une séquence peptidique de deux acides aminés et Ξ₂ représente un acide aminé, ou une séquence de formule (IV), dans laquelle Ξ₁ représente trois acides aminés, ou une séquence peptidique de formule (VIII), dans laquelle Ξ₁ représente une séquence peptidique de neuf, huit ou trois acides aminés et Ξ₂ une séquence peptidique de cinq acides aminés,
- Θ représente une séquence peptidique de formule:

   -Lys Gly Arg Leu Val-,

   ou

   - Arg Gly Arg Leu Val-,

   et
- Ω représente un groupe hydroxyle, la séquence peptidique (XV) ou une des séquences suivantes qui correspondent à la séquence peptidique de formule (XII) :

   - Val Arg Trp Asn Glu Thr-Ψ,

   - Val Gln Trp Asn Glu Thr-Ψ

   ou

   - Val Gln Trp Asn Ser Thr-Ψ.

De préférence Z représente une séquence peptidique de formule :
- -Leu Leu Ser Ser-
- -Leu Leu Ser Leu-
- -Leu Leu Asn Ser-
- -Arg Leu Asn Ser-
- -Ala Leu Glu Thr Leu Leu Gln Asn Gln Gln Leu Leu Asn Ser-
- -Ala Leu Glu Thr Leu Leu Gln Asn Gln Gln Leu Leu Asp Leu-
- -Ala Leu Glu Thr Leu Leu Gln Asn Gln Gln Leu Leu Asn Ile-
- -Leu Asn Gln Gln Arg Leu Leu Asn Ser-
   ou
- -Arg Ala Leu Glu Thr Leu Leu Asn Gln Gln Arg Leu Leu Asn Ser-

Font également partie de l'invention, les peptides synthétiques comportant de 20 à 50 acides aminés et répondant à la formule (Ia) :

Δ-Zₐ-TrpGlyCys-Θ-CysTyrThrSer-Ωₐ (Ia)

dans laquelle Zₐ représente un radical de formules IIa à Xa :

Ξ₁ₐ-Ser-Ξ₂ₐ (IIa)

-Ser-Ξ₂ₐ (IIIa)

-Ξ₁ₐ-Ser (IVa)

Ξ₁ₐ-Gln-Ξ₂ₐ (Va)

-Gln-Ξ₂ₐ (VIa)

Ξ₁ₐ-Gln- (VIIa)

Ξ₁ₐ-Asn-Ξ₂ₐ (VIIIa)

-Asn-Ξ₂ₐ (IXa)

-Ξ₁ₐ-Asn (Xa)

dans lesquelles :
- Ξ₁ₐ représente une séquence peptidique de 1 à 5 acides aminés
   et
- Ξ₂ₐ un acide aminé,
- Ωₐ représente une séquence peptidique de formule (XII), telle que définie pour la formule (I), ou une séquence peptidique de formule (XVIIa):

   Val-Σ-Zₐ-TrpGlyCys-Θ-CysTyrThrSerVal-Σ-Ψ (XVIIa)
dans laquelle Zₐ a la définition donnée pour la formule (Ia)
et
Δ, Θ, Σ et Ψ ont la même signification que pour la formule (I).

On préfère les peptides de formule (I) ou (Ia) incluant l'une des séquences suivantes (ces peptides peuvent être de type dimère ou de type monomère comme défini précédemment). Les séquences sont données selon les nomenclatures à une et à trois lettres :
Séquence N° 2
   -LLSLWGCRGRLVCYTSVQWNET-
   ou
Séquence N° 3
   -LLSSWGCKGRLVCYTSVQWNET-
   ou
Séquence N° 4
   -LLSSWGCKGRLVCYTSVQWNST-
   ou
Séquence N° 5
   -LLQSWGCKGRLVCYTSVQWNST-
   ou
Séquence N° 8
   -LLSSWGCRGRLVCYTSVQWNET-
   ou
Séquence N° 9 :
   -LLSSWGCKGRLVCYTS-
   ou
Séquence N° 10 :
   -LLNSWGCKGRLVCYTS-
   ou
Séquence N° 11 :
   -ALETLLQNQQLLNSWGCRGRLVCYTSVRWNET-
   ou
Séquence N° 12 :
   -ALETLLQNQQLLNIWGCRGRLVCYTSVRWNET-
   ou
Séquence N° 13 :
   -ALETLLQNQQLLDLWGCRGRLVCYTSVRWNET-
   ou
Séquence N° 14 :
   -LNQQRLLNSWGCKGRLVCYTSV-
   ou
Séquence N° 15 :
   -RALETLLNQQRLLNSWGCKGRLVCYTSV-
   ou
Séquence N° 16 :
   -RLNSWGCKGRLVCYTSV-
   ou
Les peptides synthétiques ci-après sont des peptides particulièrement préférés :
PEPTIDE N**°** 2 (3B) : SEQ ID N° 2
   LLSLWGCRGRLVCYTSVQWNET
   ou
PEPTIDE N° 3 (4B) : S-EQ ID N° 3
   LLSSWGCKGRLVCYTSVQWNET
   ou
PEPTIDE N° 4 (5B): SEQ ID N° 4
   LLSSWGCKGRLVCYTSVQWNST
   ou
PEPTIDE N° 5 (6B) : SEQ ID N° 5
   LLQSWGCKGRLVCYTSVQWNST
   ou
PEPTIDE N° 8 (7B) : SEQ ID N° 8
   LLSSWGCRGRLVCYTSVQWNET
   ou
PEPTIDE N° 9(12B) : SEQ ID N° 9
   LLSSWGCKGRLVCYTS
   ou
PEPTIDE N° 10(14B) : SEQ ID N° 10
   LLNSWGCKGRLVCYTS
   ou
PEPTIDE N° 11 (18B) : SEQ ID N° 11
   ALETLLQNQQLLNSWGCRGRLVCYTSVRWNET
   ou
PEPTIDE N° 12 (19B) : SEQ ID N°12
   ALETLLQNQQLLNIWGCRGRLVCYTSVRWNET
   ou
PEPTIDE N° 13 (20B) : SEQ ID N° 13
   ALETLLQNQQLLDLWGCRGRLVCYTSVRWNET
   ou
PEPTIDE N° 14 (21B) : SEQ ID N° 14
   LNQQRLLNSWGCKGRLVCYTSV
   ou
PEPTIDE N° 15 (22B) : SEQ ID N° 15
   RALETLLNQQRLLNSWGCKGRLVCYTSV
   ou
PEPTIDE N° 16(23B) : SEQ ID N° 16
   RLNSWGCKGRLVCYTSV
   ou

Les peptides synthétiques de formule (I), objet de la présente invention, peuvent être obtenus par synthèse en phase solide selon des méthodes classiques: R.B. Merrifield, *J*. *Amer. Chem. Soc. (1963), 85, pp. 2149-2154 ;* R.C. Sheppard, *in « Peptides 1971 », Nesvadba H. (ed.) North Holland, Amsterdam, pp. 111 ;* E. Atherton and R.L. Sheppard, *in « Solid phase peptide synthesis, a practical approach », IRL PRESS, (1989), Oxford University Press, pp. 25-34.* Comme synthétiseur automatique, on peut utiliser le synthétiseur « 9050 Plus Pep Synthesizer » de Millipore ou un synthétiseur équivalent.

Le support solide, utilisé pour les synthèses, doit être compatible avec la technique et la chimie utilisées. Par exemple, pour une synthèse sur le synthétiseur « 9050 Plus pep. Synthesizer », il est recommandé d'utiliser une résine adaptée à la technique dite « en flux continu » ; les résines PEG PS répondent à ces critères. Ces supports sont constitués d'un bras (« spacer ») à base de polyéthylène glycol (PEG) situé entre le groupement fonctionnel des billes de polystyrène et le point d'accrochage du premier acide aminé. La nature de ce point d'ancrage peut varier selon la fonction C-terminale choisie. Dans le cas présent, différentes résines PEG-PS ont été utilisées.

La résine de départ et les acides aminés utilisés comme matières premières sont des produits disponibles dans le commerce (PerSeptive-Biosystem, ou Néosystem).

Pour la synthèse peptidique, les groupements protecteurs de chaînes latérales suivants ont été utilisés :

| **Acides aminés** | **Groupement protecteur** |
|---|---|
| Arginine | Pentaméthyl-2,2,4,6,7-dihydrobenzofurane-5-sulfonyle (Pbf) |
| Asparagine, Glutamine | Trityle (Trt) |
| Cystéine | Trityle (Trt) ou Acétamidométhyle (Acm) |
| Sérine, Thréonine, Tyrosine | Ether tert-butyle (tBu) |
| Lysine, Tryptophane | Tert-butyloxycarbonyle (Boc) |

La protection temporaire de la fonction amine primaire en α des acides aminés utilisée est le groupement 9-fluorénylméthyloxylcarbonyle (Fmoc). La déprotection est effectuée par une solution de pipéridine à 20% en diméthylformamide.

Pour le couplage, on utilise de préférence un excès de diisopropylcarbodiimide (DIPCDI) et d'hydroxy-1-benzotriazole (HOBt).

Après synthèse, la résine est lavée avec des solvants organiques, (diméthylformamide, puis dichlorométhane) séchée sous vide puis traitée par une solution à base d'acide trifluoroacétique (TFA) refroidie à 0°C et contenant des « scavengers » appropriés. On peut utiliser, par exemple, le réactif K contenant 82% d'acide trifluoroacétique, 5% de phénol, 5% d'eau, 5% de thioanisole et 3% d'éthanedithiol.

Après filtration de la résine, les peptides synthétiques sont précipités et rincés à l'éther.

Les peptides synthétiques sont ensuite purifiés par chromatographie liquide en phase inverse et leur pureté est déterminée par spectrométrie de masse. Comme phase solide, on peut utiliser, par exemple, la phase Bondapak C-18. Les peptides sont élués en réalisant un gradient linéaire entre deux solutions tampons, le premier essentiellement aqueux (par exemple eau-TFA 0,1 %) et le second plutôt organique (par exemple un mélange contenant d'acétonitrile 60 %, eau 39,92 % et TFA 0,08 %). Les fractions pures collectées sont rassemblées, concentrées sous vide et lyophilisées.

Pour la cyclisation, les peptides synthétiques purifiés sont dissous dans une solution d'acétate d'ammonium (10mM). Le pH est ajusté à 8,5 par addition d'ammoniaque 1 M. La solution est vigoureusement agitée. La cyclisation est complète après 18 heures. Le pH est ensuite abaissé à 6 par addition d'acide acétique. Les peptides cyclisés sont lyophilisés, puis purifiés par chromatographie liquide en phase inverse comme cela a été décrit précédemment.

L'immunoréactivité des peptides de l'invention a été évaluée à l'aide de sérums de malades majoritairement d'origine camerounaise infectés par des rétrovirus VIH-1 du groupe O. Les différents essais effectués ont démontré que les peptides de l'invention, seuls ou en association (compositions de peptides), permettent de détecter 100% des sérums infectés par des rétrovirus VIH-1 du groupe O.

Les peptides synthétiques de l'invention trouvent donc leur application dans les tests immunologiques pour le dépistage des infections dues aux rétrovirus VIH-1 groupe O. Il est également possible d'utiliser des associations de plusieurs peptides synthétiques de formule I. Ces associations, qui peuvent contenir deux ou plusieurs peptides de formule I, font aussi partie de l'invention. On préfère des associations contenant les peptides N°1(2B) et N°3(4B).

Il est également possible d'utiliser des peptides synthétiques de formule (I) de la présente invention en association avec des peptides recombinés (protéines recombinantes) VIH-1 du groupe O tels qu'ils peuvent être obtenus par des méthodes classiques et ayant les séquences décrites par exemple dans la demande EP 0 591 914. De telles compositions entrent aussi dans le cadre de la présente invention.

Les peptides synthétiques de l'invention peuvent être également utilisés en association avec d'autres peptides synthétiques ou recombinés VIH-1 (protéines recombinantes) et/ou VIH-2, tels que les peptides décrits dans les demandes de brevets ou brevets EP 0 387 914, EP 0 239 425, EP 0 220 273, ou EP 0267 802. Cette liste de demandes de brevets ou brevets n'est pas exhaustive et est donnée à titre d'exemple.

Les compositions contenant un ou plusieurs peptides synthétiques de formule (I) et un ou plusieurs peptides synthétiques ou recombinés VIH-1 ou VIH-2 trouvent leur application en diagnostic pour le dépistage de malades infectés par différents rétrovirus VIH. Ces compositions font également partie de la présente invention.

Des procédés d'immunodosage in vitro utilisant un ou plusieurs peptides synthétiques de formule (I), seuls ou en association avec des peptides recombinés VIH-1 du groupe O ou des peptides synthétiques ou recombinés VIH-1 et/ou VIH-2, font également partie de l'invention.

L'invention vise également des trousses, pour la mise en oeuvre d'immunodosages, qui incluent un peptide de formule (I) ou une composition qui contient au moins un peptide de formule (I).

Les exemples suivants illustrent l'invention et sont donnés à titre non limitatif.

### EXEMPLE 1 :

### Préparation d'un composé selon l'invention ; PEPTIDE N° 2 (3B)

LLSLWGCRGRLVCYTSVQWNET
ou

Ce peptide a été synthétisé en phase solide. La technique mise au point en 1963 par Merrifield *(J. Am. Chem. Soc. (1963) 85, pp. 2149-2154)* consiste à fixer le premier acide aminé sur un support solide polymérique (résine) par sa fonction acide et à allonger la séquence peptidique à partir de ce premier acide aminé, le peptide en cours de synthèse restant ancré sur la résine.

Pour la synthèse du peptide N° 2, ont été utilisés, comme synthétiseur, le synthétiseur « 9050 Plus Pep Synthetizer » et comme résine, la résine Fmoc Thr (OtBu) PEG PS.

Les différentes étapes de la synthèse sont résumées dans le tableau I ci-après :

**Tableau I**

| **RESIDU ACIDE AMINE** | **PROTECTION NH**_{**2**} | **PROTECTION LATERALE** | **METHODE DE COUPLAGE** | **NOMBRE D'EQ - DUREE DE COUPLAGE** |
|---|---|---|---|---|
| Glu | Fmoc | OtBu | DIPCDI/HOBt | 5 eq - 30 min |
| Asn | Fmoc | Trt | DIPCDI/HOBt | 5 eq - 30 min |
| Trp | Fmoc | Boc | DIPCDI/HOBt | 5 eq - 30 min |
| Gln | Fmoc | Trt | DIPCDI/HOBt | 5 eq - 30 min |
| Val | Fmoc | | DIPCDI/HOBt | 5 eq - 30 min |
| Ser | Fmoc | tBu | DIPCDI/HOBt | 5 eq - 30 min |
| Thr | Fmoc | tBu | DIPCDI/HOBt | 5 eq - 30 min |
| Tyr | Fmoc | tBu | DIPCDI/HOBt | 5 eq - 30 min |
| Cys | Fmoc | Trt | DIPCDI/HOBt | 5 eq - 30 min |
| Val | Fmoc | | DIPCDI/HOBt | 5 eq - 30 min |
| Leu | Fmoc | | DIPCDI/HOBt | 5 eq - 30 min |
| Arg | Fmoc | Pbf | DIPCDI/HOBt | 5 eq - 30 min |
| Gly | Fmoc | | DIPCDI/HOBt | 5 eq - 30 min |
| Arg | Fmoc | Pbf | DIPCDI/HOBt | 5 eq - 30 min |
| Cys | Fmoc | Trt | DIPCDI/HOBt | 5 eq - 30 min |
| Gly | Fmoc | | DIPCDI/HOBt | 5 eq - 30 min |
| Trp | Fmoc | Boc | DIPCDI/HOBt | 5 eq - 30 min |
| Leu | Fmoc | | DIPCDI/HOBt | 5 eq - 30 min |
| Ser | Fmoc | tBu | DIPCDI/HOBt | 5 eq - 30 min |
| Leu | Fmoc | | DIPCDI/HOBt | 5 eq - 30 min |
| Leu | Fmoc | | DIPCDI/HOBt | 5 eq - 30 min |

Après la fin de la synthèse, la résine a été lavée avec du diméthylformamide, puis du dichlorométhane et séchée sous vide.

Ensuite, la résine a été traitée avec du réactif K (acide trifluoacétique 82 %; phénol 5 % ; eau 5 % ; thioanisole 5 %; éthanedithiol 3 %). Le peptide N° 2 (38), isolé par précipitation à l'aide de diéthyle oxyde, a été ensuite rincé avec le même solvant. On a ainsi obtenu 140 mg du peptide N° 2 (3B).

Le peptide N° 2 (3B) a été ensuite purifié par chromatographie liquide en phase inverse. Comme phase solide, il a été utilisé la phase Bondapak C-18. Le peptide a été élué en réalisant un gradient linéaire entre deux solutions tampons, le premier essentiellement aqueux (par exemple eau-TFA 0,1 %) et le second plutôt organique (par exemple un mélange contenant : acétonitrile 60 %, eau 39,92 % et TFA 0,08 %). Les fractions pures collectées ont été rassemblées, concentrées sous vide et lyophilisées.

Pour la cyclisation, le peptide synthétique purifié, ainsi obtenu a été dissous dans une solution d'acétate d'ammonium (10mM). Le pH a été ajusté à 8,5 par addition d'ammoniaque 1 M. La solution a été vigoureusement agitée. La cyclisation a été complète après 18 heures. Le pH a été ensuite abaissé à 6 par addition d'acide acétique. Le peptide cyclisé a été lyophilisé, puis purifié par chromatographie liquide en phase inverse comme cela a été décrit précédemment.

### Préparation d'un composé selon l'invention : PEPTIDE N° 15 (22B)

Ce peptide a été synthétisé comme le peptide N° 2 (3B), mais en utilisant comme résine, la résine FmocPAL PEG-PS.

Les différentes étapes de la synthèse sont résumées dans le tableau II ci-après :

**Tableau II**

| **RESIDU ACIDE AMINE** | **PROTECTION NH**_{**2**} | **PROTECTION LATERALE** | **METHODE DE COUPLAGE** | **NOMBRE D'EQ - DUREE DE COUPLAGE** |
|---|---|---|---|---|
| Val | Fmoc | | DIPCDI/HOBt | 5 eq - 45 mn |
| Ser | Fmoc | tBu | DIPCDI/HOBt | 5 eq - 45 mn |
| Thr | Fmoc | tBu | DIPCDI/HOBt | 5 eq - 45 mn |
| Tyr | Fmoc | tBu | DIPCDI/HOBt | 5 eq - 45 mn |
| Cys | Fmoc | Trt | DIPCDI/HOBt | 5 eq - 45 mn |
| Val | Fmoc | | DIPCDI/HOBt | 5 eq - 45 mn |
| Leu | Fmoc | | DIPCDI/HOBt | 5 eq - 45 mn |
| Arg | Fmoc | Pbf | DIPCDI/HOBt | 5 eq - 45 mn |
| Gly | Fmoc | | DIPCDI/HOBt | 5 eq - 45 mn |
| Lys | Fmoc | Boc | DIPCDI/HOBt | 5 eq - 45 mn |
| Cys | Fmoc | Trt | DIPCDI/HOBt | 5 eq - 45 mn |
| Gly | Fmoc | | DIPCDI/HOBt | 5 eq - 45 mn |
| Trp | Fmoc | Boc | DIPCDI/HOBt | 5 eq - 45 mn |
| Ser | Fmoc | tBu | DIPCDI/HOBt | 5 eq - 45 mn |
| Asn | Fmoc | Trt | DIPCDI/HOBt | 5 eq - 45 mn |
| Leu | Fmoc | | DIPCDI/HOBt | 5 eq - 45 mn |
| Leu | Fmoc | | DIPCDI/HOBt | 5 eq - 45 mn |
| Arg | Fmoc | Pbf | DIPCDI/HOBt | 5 eq - 45 mn |
| Gln | Fmoc | Trt | DIPCDI/HOBt | 5 eq - 45 mn |
| Gln | Fmoc | Trt | DIPCDI/HOBt | 5 eq - 45 mn |
| Asn | Fmoc | Trt | DIPCDI/HOBt | 5 eq - 45 mn |
| Leu | Fmoc | | DIPCDI/HOBt | 5 eq - 45 mn |
| Leu | Fmoc | | DIPCDI/HOBt | 5 eq - 45 mn |
| Thr | Fmoc | tBu | DIPCDI/HOBt | 5 eq - 45 mn |
| Glu | Fmoc | OtBu | DIPCDI/HOBt | 5 eq - 45 mn |
| Leu | Fmoc | | DIPCDI/HOBt | 5 eq - 45 mn |
| Ala | Fmoc | | DIPCDI/HOBt | 5 eq - 45 mn |
| Arg | Fmoc | Pbf | DIPCDI/HOBt | 5 eq - 45 mn |

Après la fin de la synthèse, la résine a été lavée avec du diméthylformamide, puis du dichlorométhane et séchée sous vide.

Ensuite, la résine a été traitée avec du réactif K (acide trifluoracétique 82 % ; phénol 5 % ; eau 5 % ; thioanisole 5 % ; éthanedithiol 3 %). Le peptide N°7 (22B) isolé par précipitation à l'aide d'oxyde de diéthyle, a été ensuite rincé avec le même solvant. On a ainsi obtenu 140 mg du peptide N° 15 (22B).

Le peptide N° 15 (22B) a été ensuite purifié par chromatographie liquide en phase inverse, puis cyclisé, lyophilisé et purifié comme décrit précédemment pour le peptide N° 2 (3B).

De manière équivalente, et en utilisant les résines et acides aminés appropriés, les autres composés de l'invention ont été synthétisés.

Le tableau III indique le poids moléculaire de certains peptides de formule (I), sous forme non cyclisée, évalué par spectrométrie de masse.

**Tableau III**

| **Peptide N°** | **Poids moléculaire (Daltons)** |
|---|---|
| 1 (2B) | 2512 |
| 2 (3B) | 2583 |
| 3 (4B) | 2528 |
| 4 (5B) | 2586 |
| 5 (6B) | 2527 |
| 9 (12B) | 1772. |
| 10 (14B) | 1799 |
| 11 (18B) | 3752 |
| 12 (19B) | 3778 |
| 13 (20B) | 3780 |
| 14 (21B) | 2538 |
| 15 (22B) | 3222 |
| 16 (23B) | 1941 |

### EXEMPLE 2 :

### Evaluation de l'immunoréactivité des peptides selon l'invention par test immuno-enzymatique : Essai n° 1

Les sérums utilisés ESS, DUR, VAU et HAD sont des sérums de malades français infectés par des rétrovirus VIH-1 du groupe O. Les autres échantillons de sérums de malades infectés par des rétrovirus VIH-1 groupe O ont été obtenus par le Centre Pasteur de Yaoundé au Cameroun et ont été sérotypés groupe O selon l'algorithme sérologique décrit dans *AIDS (1977), 11, pp 445-453.*

Les sérums VIH négatifs (n=48) ont été obtenus à partir de volontaires sains.

Les peptides synthétiques utilisés ont été dissous dans l'eau à une concentration de 1 mg/ml (solution mère). Pour l'étape de sensibilisation de la phase solide (coating), 110 µl d'une solution à 2 µg/ml de chaque peptide (obtenue par dilution de la solution mère avec de la solution tampon carbonate 0,1 M) ont été ajoutés à chaque cupule des plaques de microtitrage Microtiter^{™} (NUNC). Après incubation pendant une nuit à température ambiante, les microplaques ont été d'abord lavées avec une solution tampon Tris NaCl pH 7,4 contenant 0,1% de Tween® 20 et du merthiolate de sodium 0,001%, puis saturées avec une solution de PBS contenant 0,5% de Régilait^{™} (lait écrémé desséché). Après aspiration de la solution de saturation, les plaques ont été chauffées pendant 10 min à 50°C.

Les échantillons de sérums ont été dilués au 1/5 avec une solution de lait écrémé (tampon citrate additionné de 0,01% de rouge de phénol, de chloroforme à 0,25% et de Kathon® à 0,25%), déposés sur les cupules des plaques et mis à incuber pendant 30 min à 40°C.

Après lavage avec une solution tampon Tris NaCl pH 7,4 contenant 0,1 % de Tween® 20 et du merthiolate de sodium à 0,001%, 100 µl d'une solution de conjugué anticorps de chèvre anti IgG et IgM humaines marqués à la peroxydase du raifort, contenant comme conservateur 0,01% de merthiolate de sodium, en solution dans une solution tampon citrate additionné de glycérol à 30% et du sérum normal de veau foetal à 25% ont été ajoutés à chaque cupule de plaques puis ces dernières ont été mises à incuber pendant 30 min à 40°C.

Après lavage avec une solution tampon Tris NaCl pH 7,4 contenant 0,1% de Tween® 20 et du merthiolate de sodium à 0,001%, le développement de la coloration a été obtenu par addition, dans chaque cupule, de 100 µl de O-phenylène diamine en solution dans du peroxyde d'hydrogène. Les microplaques ont été ensuite mises à incuber pendant 30 min à température ambiante et dans l'obscurité. La réaction colorée a ensuite été arrêtée par addition de 100 µl d'acide sulfurique 4N. L'absorbance (A) a été déterminée à 490 et 620 nm.

L'absorbance relative (A490-A620) lue dans chaque cupule est proportionnelle à l'immunoréactivité de chaque peptide. Cela indique l'aptitude de chaque peptide à réagir avec l'échantillon biologique avec lequel est effectué l'essai. La valeur seuil (cut-off) a été déterminée comme étant une absorbance égale à 0.15. Elle correspond à la moyenne de valeurs négatives (n=48) plus 12 écart-types.

La réactivité des peptides de l'invention. (peptide N° 3 (4B) et peptide N° 2 (3B)) tous sous forme cyclisée) a été comparée à celle de deux peptides synthétiques ayant comme séquence une partie de la séquence naturelle de l'enveloppe (env) de l'isolat VAU (rétrovirus VIH-1 du groupe O) et comportant un épitope immunodominant de gp41.

Ces deux peptides ont la séquence suivante :

Pour l'étude, ces peptides ont été utilisés sous forme cyclisée. Les résultats de cette étude sont indiqués dans le tableau IV.

**Tableau IV**

| **SERUM** | **ABSORBANCE** | | | |
|---|---|---|---|---|
| | **PEPTIDE N° 3 (4B)** | **PEPTIDE N° 2 (3B)** | **VAU 22 AA** | **VAU 35 AA** |
| **ESS*** | >** | > | > | > |
| **DUR*** | > | > | 0.118 | 0,872 |
| **HAD** | > | 0,518 | 0,789 | 0.871 |
| **VAU*** | 1,342 | > | > | > |
| **3935** | > | 0,893 | 0,138 | 0,227 |
| **6891** | > | 0,614 | 0,359 | 0,496 |
| **6512*** | 0,746 | 0,785 | 0,120 | 0,174 |
| **1105*** | 1,421 | 1,031 | 0,099 | 0,129 |
| **4021*** | 0,430 | 0,119 | 0,050 | 1,957 |
| **5969*** | > | 0,282 | 2,491 | > |
| **2700** | > | 0,274 | > | > |
| **5453** | 0,555 | 0,081 | 1,267 | 1,482 |
| **5931** | > | > | 0,202 | 2,225 |
| **3136** | > | 0,992 | > | > |
| **3653** | 1,352 | > | 1,441 | 1,322 |
| **2352** | > | > | > | > |
| **3016** | > | > | > | > |
| **3302** | > | > | > | > |
| **2294** | > | > | > | > |
| **3771** | > | > | > | > |
| **1581** | > | > | 1,112 | 0,894 |
| **5373** | > | > | 1,359 | 0,856 |
| **7443** | > | > | 0,920 | 0,574 |
| **3637** | > | > | 0,779 | 1,647 |
| **6295*** | 1,718 | 1,063 | 0,972 | > |
| **6689*** | 0,710 | > | > | > |
| **1754** | > | > | 1,263 | 1,948 |
| **4489*** | > | > | 1,318 | 1,718 |
| **4364** | > | > | > | > |
| **3884*** | > | > | > | > |
| **3529** | > | > | > | > |
| **3482** | 2,402 | > | > | > |
| **1702** | > | > | > | > |
| **6487** | > | 1,017 | 2,889 | 2,891 |
| **5164** | > | > | > | > |
| **5766*** | > | > | > | > |
| **3945** | > | > | > | > |
| **4434** | > | > | 2,273 | > |
| **4288*** | > | > | 2,337 | N.T.*** |
| **6782** | > | 2,091 | 2,190 | 2,214 |
| **2313** | > | > | > | > |
| **2312** | > | > | > | > |
| **1062** | > | > | > | > |
| **402** | > | > | > | > |
| **134** | > | > | > | > |
| **7120** | > | > | > | > |
| **7212** | > | > | > | > |
| **6976*** | > | > | > | > |
| **3600*** | > | > | > | > |
| **3236** | > | > | > | > |
| **3235** | > | > | > | > |
| **2551** | > | > | > | > |
| **5270*** | > | > | > | > |
| **5210** | > | > | > | > |
| **5149*** | > | > | > | > |
| **4477** | > | > | 2,511 | > |
| **3891** | > | > | > | > |
| **3627*** | > | > | > | > |
| **7258*** | > | > | > | > |
| **7007** | 2,136 | 2,334 | > | 2,151 |
| **6697** | > | > | > | > |
| **6998** | > | > | > | > |
| **6627** | > | > | > | > |
| **6198*** | > | > | > | > |
| **6165** | > | > | > | > |
| **7439** .. | > | > | > | > |
| **7297*** | > | > | > | > |
| **6111** | > | > | > | > |
| **625** | > | > | > | 2, 885 |

| | | | | |
|---|---|---|---|---|
| * sérotypés / génotypés ** > = signal supérieur à la capacité de lecture du spectrophotomètre. *** Non testé | | | | |

Les résultats du tableau IV démontrent que le peptide N° 3 (4B) présente les meilleures performances au regard de celles relevées pour les autres peptides. Ce peptide permet la meilleure discrimination des sérums de malades infectés par des rétrovirus VIH-1 du groupe O par rapport aux deux peptides ayant une partie de la séquence de l'isolat VAU correspondant à l'épitope immunodominant de la gp41. Par ailleurs, le peptide N° 2 (3B) de l'invention est plus immunoréactif que le peptide VAU 22 AA qui comporte le même nombre d'acides aminés.

### EXEMPLE 3 :

### Evaluation par test immunoenzymatique de l'immunoréactivité des peptides selon l'invention : Essai n° 2

Les échantillons de sérums de malades infectés par des rétrovirus VIH-1 du groupe O ont été obtenus par le Centre Pasteur de Yaoundé au Cameroun et ont été serotypés groupe O selon l'algorithme sérologique décrit dans *AIDS (1977), 11, pp. 445-453.* Un échantillon (Maryland) génotypé provient des Etats-Unis. Ces échantillons ont été préalablement dilués en sérum humain négatif aux dilutions données dans le tableau V, afin de disposer d'un volume suffisant pour les différents essais d'immunoréactivité.

Les peptides synthétiques utilisés ont été dissous dans l'eau à une concentration de 1 mg/ml (solution mère). Pour l'étape de sensibilisation de la phase solide (« coating »), il a été procédé comme cela a été décrit pour l'exemple 2.

Les échantillons de sérums ont été dilués au 1/5 avec une solution de lait écrémé (en tampon citrate additionné de rouge de phénol à 0,01%, de chloroforme à 0,25% et de Kathon® à 0,25%), déposés dans les cupules des plaques et mis à incuber pendant 30 min à 40°C.

Après lavage avec une solution tampon Tris NaCl pH 7,4 contenant 0,1% de Tween® 20 et du merthiolate de sodium à 0,001%, 100 µl d'une solution de conjugué d'anticorps de chèvre anti IgG et IgM humaines marqués à la peroxydase du raifort, contenant comme conservateur du merthiolate de sodium à 0,01%, en solution dans une solution tampon citrate additionnée de glycérol à 30% et du sérum normal de veau foetal à 25%, ont été ajoutés à chaque cupule des plaques puis ces dernières ont été mises à incuber pendant 30 mn à 40°C.

Après lavage avec une solution tampon Tris NaCl pH 7,4 contenant du Tween® 20 à 0,1% et du merthiolate de sodium à 0,001%, le développement de la coloration a été obtenu comme cela a été décrit dans l'exemple 2.

L'absorbance (DO) relative (A490-A620) lue dans chaque cupule est proportionnelle à l'immunoréactivité de chaque peptide. Cela indique l'aptitude de chaque peptide à réagir avec l'échantillon biologique avec lequel est effectué l'essai.

La réactivité des peptides de l'invention, peptides N° 10 (14B), N° 11 (18B), N° 12 (19B), N° 14 (21B), N° 15 (22B), N° 16 (23B) tous sous forme cyclisée, a été comparée à celle de trois peptides synthétiques homologues, ayant comme séquence une partie de là séquence naturelle de l'enveloppe (env) de rétrovirus VIH-1 du groupe O. Ces peptides sont deux peptides dérivés de l'isolat VAU, -le peptide VAU 22 AA et le peptide VAU 35 AA- et le peptide MVP 5180 (désigné « MVP 5180 » dans le tableau V). Les peptides VAU 22 AA et VAU 35 AA (dont la structure est indiquée dans l'exemple 2) et le peptide MVP 5180 comportent un épitope immunodominant de la gp41.

Tous ces peptides ont été utilisés sous forme cyclisée. La séquence du peptide MVP 5180 est la suivante :

Les résultats de cette étude sont indiqués dans le tableau V.

**Tableau V**

| **SERUM** | **PEPTIDES*** | | | | | | | | |
|---|---|---|---|---|---|---|---|---|---|
| | **N° 10** | **N° 11** | **N° 12** | **N° 14** | **N° 15** | **N° 16** | **MVP 5180** | **VAU 35 AA** | **VAU 22 AA** |
| | **ABSORBANCE (DO)** | | | | | | | | |
| **4280** | 0,022 | 0,686 | 0,201 | 0,286 | 0,689 | 0,033 | 0,382 | 0,013 | 0,021 |
| **au 1/50** | | | | | | | | | |
| **NGO** | 0,067 | 0,335 | 0,193 | 0,157 | 0,315 | 0,110 | 0,184 | 0,055 | 0,040 |
| **au 1/50** | | | | | | | | | |
| **NJEM** | 0,032 | 0,811 | 0,391 | 0,277 | 0,939 | 0,025 | 0,146 | 0,159 | 0,024 |
| **au 1/100** | | | | | | | | | |
| **MBASSI** | 1,217 | 1,150 | 0,747 | 2,134 | 2,010 | 2,683 | 0,248 | 0,120 | 0,257 |
| **au 1/100** | | | | | | | | | |
| **WANG** | 0,698 | 0,234 | 0,124 | 2,397 | 2,680 | 1,290 | 0,075 | 0,025 | 0,041 |
| **au 1/50** | | | | | | | | | |
| **258 OUDI** | 0,587 | 0,373 | 0,226 | 0,764 | 1,184 | 1,692 | 0,116 | 0,058 | 0,100 |
| **au 1/100** | | | | | | | | | |
| **DO15** | 1,613 | 0,859 | 1,286 | 3,357 | 3,693 | 3,038 | 0,673 | 0,036 | 0,075 |
| **au 1/100** | | | | | | | | | |
| **DJOU** | 1,268 | 0,482 | 0,419 | 1,998 | 2,088 | 2,166 | 0,203 | 0,022 | 0,042 |
| **au 1/100** | | | | | | | | | |
| **3600** | 0,482 | 0,360 | 0,249 | 0,716 | 0,801 | 0,933 | 0,206 | 0,025 | 0,058 |
| **au 1/100** | | | | | | | | | |
| **3613** | 1,108 | 0,837 | 0,773 | 1,508 | 1,627 | 1,679 | 0,478 | 0,250 | 0,396 |
| **au 1/400** | | | | | | | | | |
| **6111** | 0,596 | 0,348 | 0,202 | 0,850 | 1,207 | 1,009 | 0,226 | 0,087 | 0,180 |
| **au 1/100** | | | | | | | | | |
| **625** | 0,838 | 0,338 | 0,264 | 2,045 | 2,122 | 1,791 | 0,202 | 0,069 | 0,165 |
| **au 1/50** | | | | | | | | | |
| **Maryland** | 0,524 | 0,370 | 0,285 | 0,734 | 0,844 | 1,229 | 0,241 | 0,054 | 0,168 |
| **au 1/400** | | | | | | | | | |
| **3653** | 0,347 | 0,337 | 0,247 | 0,072 | 0,380 | 0,406 | 0,401 | 0,021 | 0,310 |
| **au 1/10** | | | | | | | | | |

| | | | | | | | | | |
|---|---|---|---|---|---|---|---|---|---|
| PHASE SOLIDE*: PEPTIDE 2µg/ml | | | | | | | | | |

Pour chaque peptide testé, les échantillons ont été rangés en quatre classes (a, b, c, et d) correspondant à divers niveaux d'absorbance relative lue aux longueurs d'onde A492-A620 :
- pour a : DO < 0,100,
- pour b : 0,100 < DO < 0,500,
- pour c : 0,500 < DO < 1,000,
- pour d : DO > 1,000,
permettant ainsi d'évaluer le degré d'immunoréactivité des peptides. Les peptides les plus immunoréactifs sont ceux pour lesquels le plus grand nombre d'échantillons est trouvé dans les classes correspondant aux absorbances les plus élevées.

Les résultats sont indiqués dans le tableau VI.

**Tableau VI**

| **CLASSE** | **PEPTIDES *** | | | | | | | | |
|---|---|---|---|---|---|---|---|---|---|
| | **N° 10** | **N° 11** | **N° 12** | **N° 14** | **N° 15** | **N° 16** | **MVP 5180** | **VAU 35 AA** | **VAU 22 AA** |
| | **Nombre d'échantillons** | | | | | | | | |
| **a** | 3 | 0 | 0 | 1 | 0 | 2 | 1 | 11 | 7 |
| **b** | 2 | 9 | 11 | 3 | 2 | 2 | 12 | 3 | 7 |
| **c** | 5 | 4 | 2 | 4 | 4 | 1 | 1 | 0 | 0 |
| **d** | 4 | 1 | 1 | 6 | 8 | 9 | 0 | 0 | 0 |

| | | | | | | | | | |
|---|---|---|---|---|---|---|---|---|---|
| PHASE SOLIDE*: PEPTIDE 2µg/ml | | | | | | | | | |

Les résultats montrent que tous les peptides de l'invention testés réalisent une meilleure performance en immunoréactivité que les peptides de référence de l'art antérieur qui dérivent d'isolats naturels (MVP 5180, VAU). Les peptides de l'invention N° 15 (22B), N° 14 (21 B), et N° 16 (23B) s'avèrent les plus immunoréactifs.

### EXEMPLE 4 :

### Evaluation de l'immunoréactivité des compositions contenant des peptides selon l'invention par test immuno-enzymatique.

Pour cet essai, il a été procédé selon le protocole décrit dans l'exemple 2 et les mêmes sérums ont été utilisés. Les microplaques utilisées ont été sensibilisées avec le peptide N° 3 (4B) cyclisé. Dans chaque cupule ont été déposés, 100 µl d'une solution contenant 2 µg/ml de peptide N° 3 (4B).

Les résultats de cet essai sont donnés dans le tableau VII.

**Tableau VII**

| SERUM | ABSORBANCE |
|---|---|
| | PEPTIDE N° 3 (48) (2 µg/ml) |
| **3529** | >* |
| **1105** | 1,421 |
| **3891** | > |
| **3235** | > |
| **2700** | > |
| **5931** | > |
| **3935** | > |
| **7443** | > |
| **1062** | > |
| **1754** | > |
| **3136** | > |
| **6891** | > |
| **5149** | > |
| **5270** | > |
| **2551** | > |
| **3600** | > |
| **6976** | > |
| **4489** | > |
| **6165** | > |
| **6198** | > |
| **6627** | > |
| **6998** | > |
| **6697** | > |
| **7258** | > |
| **3627** | > |
| **4477** | > |
| **3771** | > |
| **1702** | > |
| **2294** | > |
| **2352** | > |
| **3016** | > |
| **3302** | > |
| **3482** | > |
| **3653** | 1,322 |
| **4364** | > |
| **3637** | > |
| **4288** | > |
| **5969** | > |
| **258** | > |
| **6111** | > |
| **625** | > |
| **6853** | 2.769 |
| **3136** | > |
| **6689** | 0,710 |
| **6295** | 1.718 |
| **4021** | 0.430 |
| **3884** | > |
| **6512** | 0,746 |
| **6487** | > |
| **ESS** | > |
| **HAD** | > |
| **DUR** | > |

| | |
|---|---|
| * > = signal supérieur à la capacité de lecture du spectrophotomètre. | |

Les résultats du tableau VII démontrent que les compositions des peptides de l'invention, lorsque utilisées en diagnostic permettent la détection de tous les sérums de malades infectés par des rétrovirus VIH-1 du groupe O.

## Revendications

1. Peptides synthétiques de type monomère de 13 à 33 acides aminés ou de type dimère de 26 à 66 acides aminés, sous forme linéaire ou sous forme cyclisée par l'intermédiaire de ponts disulfures inter-cystéines, répondant à la formule générale (I) :
Δ-Z-TrpGlyCys-Θ-CysTyrThrSer-Ω (I)
dans laquelle :
- Δ représente un radical biotinyle, un radical biocytinyle, un atome d'hydrogène, un radical acétyle (CH₃CO-), une chaîne aliphatique contenant éventuellement une ou deux fonctions thiol, aldéhyde ou amine,
- Z représente une séquence peptidique d'une des formules (II) à (X) :
-Ξ₁-Ser-Ξ₂- (II)
-Ser-Ξ₂- (III)
-Ξ₁-Ser- (IV)
-Ξ₁-Gln-Ξ₂- (V)
-Gln-Ξ₂- (VI)
-Ξ₁-Gln- (VII)
-Ξ₁-Asn-Ξ₂- (VIII)
-Asn-Ξ₂- (IX)
Ξ₁-Asn- (X)
dans lesquelles :
- Ξ₁, représente une séquence peptidique de 0 à 9 acides aminés
et
- Ξ₂ représente une séquence peptidique de 0 à 5 acides aminés,
- Θ représente une séquence peptidique de formule (XI) :
-(AA₁)-(AA₂)-(AA₃)-(AA₄)-(AA₅)- (XI)
dans laquelle :
• (AA₁) représente soit un résidu lysine, soit un résidu arginine, soit un résidu ornithine,
• (AA₂) représente soit un résidu glycine, soit un résidu asparagine,
• (AA₃) représente soit un résidu lysine, soit un résidu arginine, soit un résidu ornithine,
• (AA₄) représente soit un résidu leucine, soit un résidu isoleucine, soit un résidu glutamine,
• (AA₅) représente soit un résidu isoleucine, soit un résidu valine, soit un résidu leucine, soit un résidu thréonine, soit un résidu norleucine, soit un résidu norvaline,
à condition toutefois que (AA₁), (AA₂), (AA₃), (AA₄) et (AA₅) ne forment jamais ensemble les séquences peptidiques -Lys Gly Lys Leu Ile- et -Lys Gly Lys Leu Val-,
- Ω, fixé sur le groupe -CO- de la sérine représente :
- un radical hydroxyle (-OH) ou un radical amino (-NH₂),
- un radical alcoxy comportant de 1 à 6 atomes de carbone,
- une séquence peptidique de formule (XII) :
-Val-Σ-Ψ (XII)
dans laquelle Σ représente une séquence de formule (XIII) ou de formule (XIV) :
-(AA₆)-Trp Asn-(AA₇)-(AA₈) (XIII)
-(AA₆)-Trp His-(AA₇)-(AA₈) (XIV)
dans lesquelles :
• (AA₆) représente un acide aminé différent de la lysine,
• (AA₇) représente un acide aminé,
• (AA₈) représente un résidu sérine ou thréonine,
et Ψ fixé sur le reste -CO- de l'acide aminé AA₈ libre, représente un groupe OH, NH₂ ou un radical alcoxy comportant de 1 à 6 atomes de carbone,
- une séquence peptidique de formule (XV) :
-Val-Ψ (XV)
dans laquelle Ψ fixé sur le reste -CO- de la valine, a la même signification que pour la formule (XII),
- ou une séquence peptidique de formule (XVI) à (XVIII) :
-Z-TrpGlyCys-Θ-CysTyrThrSer-Ψ (XVI)
Val-Σ-Z-TrpGlyCys-Θ-CysTyrThrSerVal-Σ-Ψ (XVII)
Val-Z-TrpGlyCys-Θ-CysTyrThrSerVal-Ψ (XVIII)
dans lesquelles Z et Θ ont la définition donnée pour la formule (I) et Σ a la définition donnée pour la formule (XII) et Ψ fixé sur le reste -CO- de la sérine, sur le reste -CO- de l'acide aminé AA₈ ou sur le reste-CO- de la valine, a la même signification que pour la formule (XII).

2. Peptides synthétiques de formule (I) selon la revendication 1, dans laquelle ladite chaîne aliphatique représentée par Δ est une chaîne alkyle de 1 à 6 atomes de carbone ou une chaîne alcényle de 2 à 6 atomes de carbone, ou une chaîne aminoalkylcarbonyle de 2 à 6 atomes de carbone.

3. Peptides synthétiques de formule (I) selon la revendication 1 ou 2, dans laquelle (AA₅) représente soit un résidu valine, soit un résidu leucine, soit un résidu thréonine et lorsque Ω correspond à une séquence peptidique de formule (XII) ou (XIV), (AA₆) représente soit un résidu glutamine, soit un résidu arginine.

4. Peptides synthétiques de formule (I), selon la revendication 1 ou 2, dans laquelle :
- Δ représente un radical biotinyle, un atome d'hydrogène, ou une chaîne aliphatique contenant éventuellement une ou deux fonctions thiol, aldéhyde ou amine, la chaîne aliphatique étant une chaîne alkyle de 1 à 6 atomes de carbone, ou une chaîne aminoalkylcarbonyle de 2 à 6 atomes de carbone,
- Z représente une séquence peptidique de formule (II) ou (V) dans lesquelles Ξ₁ représente une séquence peptidique de deux acides aminés et Ξ₂ représente un acide aminé, ou une séquence de formule (IV) dans laquelle Ξ₁ représente trois acides aminés, ou une séquence peptidique de formule (VIII) dans laquelle Ξ₁ représente une séquence peptidique de neuf, huit ou trois acides aminés et Ξ₂ une séquence peptidique de cinq acides aminés,
- Θ représente une séquence peptidique de formule :
-Lys Gly Arg Leu Val-,
ou
- Arg Gly Arg Leu Val-,
et
- Ω représente un groupe hydroxyle, la séquence peptidique (XV) ou une des séquences suivantes qui correspondent à la séquence peptidique de formule (XII) :
- Val Arg Trp Asn Glu Thr-Ψ,
- Val Gln Trp Asn Glu Thr-Ψ
ou
- Val Gln Trp Asn Ser Thr-Ψ.

5. Peptides synthétiques de formule (I), selon l'une des revendications 1 à 4, dans laquelle Z représente une séquence peptidique de formule :
• -Leu Leu Ser Ser-
• -Leu Leu Ser Leu
• -Leu Leu Asn Ser-
• -Arg Leu Asn Ser-
• -Ala Leu Glu Thr Leu Leu Gln Asn Gln Gln Leu Leu Asn Ser-
• -Ala Leu Glu Thr Leu Leu Gln Asn Gln Gln Leu Leu Asp Leu-
• -Ala Leu Glu Thr Leu Leu Gln Asn Gln Gln Leu Leu Asn Ile -
• -Leu Asn Gln Gln Arg Leu Leu Asn Ser-
ou
• -Arg Ala Leu Glu Thr Leu Leu Asn Gln Gln Arg Leu Leu Asn Ser-

6. Peptides synthétiques de 20 à 50 acides aminés selon la revendication 1, de formule (Ia) :
Δ-Zₐ-TrpGlyCys-Θ-CysTyrThrSer-Ωₐ (Ia)
dans laquelle Za représente un radical de formules IIa à Xa :
Ξ₁ₐ-Ser-Ξ₂ₐ (IIa)
-Ser-Ξ₂ₐ (IIIa)
-Ξ₁ₐ-Ser (IVa)
Ξ₁ₐ-Gln-Ξ₂ₐ (Va)
-Gln-Ξ₂ₐ (VIa)
Ξ₁ₐ-Gln- (VIIa)
Ξ₁ₐ-Asn-Ξ₂ₐ (VIIIa)
-Asn-Ξ₂ₐ (IXa)
-Ξ₁ₐ-Asn (Xa)
dans lesquelles :
- Ξ₁ₐ représente une séquence peptidique de 1 à 5 acides aminés
et
- Ξ₂ₐ un acide aminé,
- Ωₐ représente une séquence peptidique de formule (XII), telle que définie pour la formule (I), ou une séquence peptidique de formule (XVIIa) :
Val-Σ-Zₐ-TrpGlyCys-Θ-CysTyrThrSerVal-Σ-Ψ (XVIIa)
et
Δ, Θ, Σ et Ψ ont la même signification que pour la formule (I).

7. Peptides synthétiques de formule (I) selon l'une des revendications 1 à 6 incluant l'une des séquences suivantes :
Séquence N° 2
-LLSLWGCRGRLVCYTSVQWNET-
ou
Séquence N° 3
-LLSSWGCKGRLVCYTSVQWNET-
ou
Séquence N° 4
-LLSSWGCKGRLVCYTSVQWNST-
ou
Séquence N° 5
-LLQSWGCKGRLVCYTSVQWNST-
ou
Séquence N° 8
-LLSSWGCRGRLVCYTSVQWNET-
ou
Séquence N° 9 :
-LLSSWGCKGRLVCYTS-
ou
Séquence N° 10 :
-LLNSWGCKGRLVCYTS-
ou
Séquence N° 11 :
-ALETLLQNQQLLNSWGCRGRLVCYTSVRWNET-
ou
Séquence N° 12 :
-ALETLLQNQQLLNIWGCRGRLVCYTSVRWNET-
ou
Séquence N° 13 :
-ALETLLQNQQLLDLWGCRGRLVCYTSVRWNET-
ou
Séquence N° 14 :
-LNQQRLLNSWGCKGRLVCYTSV-
ou
Séquence N° 15 :
-RALETLLNQQRLLNSWGCKGRLVCYTSV-
ou
Séquence N° 16 :
-RLNSWGCKGRLVCYTSV-
ou

8. Peptides synthétiques, selon l'une des revendications 1 à 7, de séquence :
PEPTIDE N° 2 (3B)
LLSLWGCRGRLVCYTSVQWNET
ou
PEPTIDE N° 3 (4B)
LLSSWGCKGRLVCYTSVQWNET
ou
PEPTIDE N° 4 (5B)
LLSSWGCKGRLVCYTSVQWNST
ou
PEPTIDE N° 5 (6B)
LLQSWGCKGRLVCYTSVQWNST
ou
PEPTIDE N° 8 (7B)
LLSSWGCRGRLVCYTSVQWNET
ou
PEPTIDE N° 9 (12B)
LLSSWGCKGRLVCYTS
ou
PEPTIDE N° 10 (14B)
LLNSWGCKGRLVCYTS
ou
PEPTIDE N° 11 (18B)
ALETLLQNQQLLNSWGCRGRLVCYTSVRWNET
ou
PEPTIDE N° 12 (19B)
ALETLLQNQQLLNIWGCRGRLVCYTSVRWNET
ou
PEPTIDE N° 13 (20B)
ALETLLQNQQLLDLWGCRGRLVCYTSVRWNET
ou
PEPTIDE N° 14 (21B)
LNQQRLLNSWGCKGRLVCYTSV
ou
PEPTIDE N° 15 (22B)
RALETLLNQQRLLNSWGCKGRLVCYTSV
ou
PEPTIDE N° 16 (23B)
RLNSWGCKGRLVCYTSV
ou

9. Composition contenant un ou plusieurs peptides synthétiques de formule (1) selon l'une quelconque des revendications 1 à 8.

10. Composition selon la revendication 9 contenant le peptide N° 3 (4B).

11. Composition contenant un ou plusieurs peptides synthétiques de formule (1) selon la revendication 1 et un ou plusieurs peptides recombinés VIH-1 du groupe O.

12. Composition contenant un ou plusieurs peptides synthétiques de formule (I), selon la revendication 1, et un ou plusieurs peptides synthétiques ou recombinés VIH-1 et/ou VIH-2.

13. Procédé d'immunodosage in vitro mettant en oeuvre un ou plusieurs peptides synthétiques de formule (I), selon l'une quelconque des revendications 1 à 8.

14. Procédé d'immunodosage in vitro mettant en oeuvre une composition selon l'une quelconque des revendications 9 à 12.

15. Trousse de diagnostic incluant au moins un peptide synthétique de formule (I), selon l'une quelconque des revendications 1 à 8, ou une composition selon l'une quelconque des revendications 9 à 12.

## Claims

1. Synthetic peptides of the monomeric type having 13 to 33 amino acids or of the dimeric type having 26 to 66 amino acids in the linear form or in the form which is cyclized by means of intercysteine disulfide bridges, corresponding to the general formula (I):
△-Z-TrpGlyCys-Θ-CysTyrThrSer-Ω (I)
in which
- Δ represents a biotinyl radical, a biocytinyl radical, a hydrogen atom, an acetyl radical (CH₃CO-), an aliphatic chain optionally containing one or two thiol, aldehyde or amine functions,
- Z represents a peptide sequence of one of the formulae (II) to (X) :
-Ξ₁-Ser-Ξ₂- (II)
-Ser-Ξ₂- (III)
-Ξ₁-Ser- (IV)
-Ξ₁-Gln-Ξ₂ (V)
-Gln-Ξ₂- (VI)
-Ξ₁-Gln- (VII)
-Ξ₁-Asn-Ξ₂- (VIII)
-Asn-Ξ₂- (IX)
-Ξ₁-Asn (X)
in which
- Ξ₁ represents a peptide sequence of 0 to 9 amino acids
and
- Ξ₂ represents a peptide sequence of 0 to 5 amino acids,
- Ω represents a peptide sequence of the formula (XI) :
-(AA₁)-(AA₂)-(AA₃)-(AA₄)-(AA₅)- (XI)
in which:
• (AA₁) represents a lysine residue or an arginine residue or an ornithine residue,
• (AA₂) represents a glycine residue or an asparagine residue,
• (AA₃) represents a lysine residue or an arginine residue or an ornithine residue,
• (AA₄) represents a leucine residue or an isoleucine residue or a glutamine residue
• (AA₅) represents an isoleucine residue or a valine residue or a leucine residue or a threonine residue or a norleucine residue or a norvaline residue
provided that, however, (AA₁), (AA₂), (AA₃), (AA₄) and (AA₅) together never form the peptide sequences -Lys Gly Lys Leu Ile- and -Lys Gly Lys Leu Val-
Ω , attached to the -CO- group of the serine, represents:
- a hydroxyl radical (-OH) or an amino radical (-NH₂),
- an alkoxy radical containing 1 to 6 carbon atoms,
- a peptide sequence of the formula (XII):
-Val-Σ-Ψ (XII)
in which Σ represents a sequence of the formula (XIII) or of the formula (XIV) :
- (AA₆)-Trp Asn-(AA₇)-(AA₈) (XIII)
- (AA₆)-Trp His-(AA₇)-(AA₈) (XIV)
in which:
• (AA₆) represents an amino acid other than lysine,
• (AA₇) represents an amino acid,
• (AA₈) represents a serine or threonine residue,
and Ψ, attached to the -CO- radical of the free amino acid AA₈, represents an OH or NH₂ group or an alkoxy radical containing 1 to 6 carbon atoms,
- a peptide sequence of the formula (XV) :
-Val-Ψ (XV)
in which Ψ, attached to the -CO- radical of the valine, has the same meaning as for the formula (XII),
- or a peptide sequence of the formula (XVI) to (XVIII):
-Z-TrpGlyCys-Θ-CysTyrThrSer-Ψ (XVI)
Val-Σ-Z-TrpGlyCys-Θ-CysTyrThrSerVal-Σ-Ψ (XVII)
Val-Z-TrpGlyCys-Θ-CysTyrThrSerVal-Ψ (XVIII)
in which Z and Θ have the meaning given for the formula (I) and Σ has the meaning given for the formula (XII) and Ψ, attached to the -CO- radical of the serine, to the -CO- radical of the amino acid AA₈ or to the -CO- radical of the valine, has the same meaning as for the formula (XII).

2. Synthetic peptides of the formula (I) according to claim 1, in which the said aliphatic chain represented by Δ is an alkyl chain of 1 to 6 carbon atoms or an alkenyl chain of 2 to 6 carbon atoms or an aminoalkylcarbonyl chain of 2 to 6 carbon atoms.

3. Synthetic peptides of the formula (I) according to claim 1 or 2, in which (AA₅) represents a valine residue or a leucine residue or a threonine residue, and if Ω corresponds to a peptide sequence of the formula (XII) or (XIV), (AA₆) represents a glutamine residue or an arginine residue.

4. Synthetic peptides of the formula (I) according to claim 1 or 2, in which:
- Δ represents a biotinyl radical, a hydrogen atom or an aliphatic chain optionally containing one or two thiol, aldehyde or amine functions, the aliphatic chain being an alkyl chain of 1 to 6 carbon atoms or an aminoalkylcarbonyl chain of 2 to 6 carbon atoms,
- Z represents a peptide sequence of the formula (II) or (V) in which Ξ₁ represents a peptide sequence of two amino acids and Ξ₂ represents an amino acid, or a sequence of the formula (IV) in which Ξ₁ represents three amino acids, or a peptide sequence of the formula (VIII) in which Ξ₁ represents a peptides sequence of nine, eight or three amino acids and Ξ₂ represents a peptide sequence of five amino acids,
- Θ represents a peptide sequence of the formula:
-Lys Gly Arg Leu Val-
or
-Arg Gly Arg Leu Val-
and
- Ω represents a hydroxyl group, the peptide sequence (XV) or one of the following sequences which correspond to the peptide sequence of the formula (XII):
-Val Arg Trp Asn Glu Thr-Ψ,
-Val Gln Trp Asn Glu Thr-Ψ,
or
-Val Gln Trp Asn Ser Thr-Ψ.

5. Synthetic peptides of the formula (I) according to one of claims 1 to 4, in which Z represents a peptide sequence of the formula:
• -Leu Leu Ser Ser-
• -Leu Leu Ser Leu-
• -Leu Leu Asn Ser-
• -Arg Leu Asn Ser-
• -Ala Leu Glu Thr Leu Leu Gln Asn Gln Gln Leu Leu Asn Ser-
• -Ala Leu Glu Thr Leu Leu Gln Asn Gln Gln Leu Leu Asp Leu-
• -Ala Leu Glu Thr Leu Leu Gln Asn Gln Gln Leu Leu Asn Ile-
• -Leu Asn Gln Gln Arg Leu Leu Asn Ser-
or
• -Arg Ala Leu Glu Thr Leu Leu Asn Gln Gln Arg Leu Leu Asn Ser-

6. Synthetic peptides having 20 to 50 amino acids according to claim 1, of the formula (Ia) :
Δ-Zₐ-TrpGlyCys-Θ-CysTyrThrSer-Ωₐ (Ia)
in which Zₐ represents a radical of the formulae IIa to Xa:
-Ξ₁ₐ-Ser-Ξ₂ₐ (IIa)
-Ser-Ξ₂ₐ- (IIIa)
-Ξ₁ₐ-Ser- (IVa)
-Ξ₁ₐ-Gln-Ξ₂ₐ (Va)
-Gln-Ξ₂ₐ- (VIa)
-Ξ₁ₐ-Gln- (VIIa)
-Ξ₁ₐ-Asn-Ξ₂ₐ- (VIIIa)
-Asn-Ξ₂ₐ- (IXa)
-Ξ₁ₐ-Asn (Xa)
in which
- Ξ₁ₐ represents a peptide sequence of 1 to 5 amino acids
and
- Ξ₂ₐ represents an amino acid,
- Ωₐ represents a peptide sequence of the formula (XII) as defined for the formula (I) or a peptide sequence of the formula (XVIIa):
Val-Σ-Zₐ-TrpGlyCys-Θ-CysTyrThrSerVal-Σ-Ψ (XVIIa)
and
Δ, Θ, Σ and Ψ have the same meaning as for the formula (I).

7. Synthetic peptides of the formula (I) according to one of claims 1 to 6 including one of the following sequences:
Sequence no. 2
-LLSLWGCRGRLVCYTSVQWNET-
or
Sequence no. 3
-LLSSWGCKGRLVCYTSVQWNET-
or
Sequence no. 4
-LLSSWGCKGRLVCYTSVQWNST-
or
Sequence no. 5
-LLQSWGCKGRLVCYTSVQWNST-
or
Sequence no. 8
-LLSSWGCRGRLVCYTSVQWNET-
or
Sequence no. 9:
-LLSSWGCKGRLVCYTS-
or
Sequence no. 10:
-LLNSWGCKGRLVCYTS-
or
Sequence no. 11:
-ALETLLQNQQLLNSWGCRGRLVCYTSVRWNET-
or
Sequence no. 12:
-ALETLLQNQQLLNIWGCRGRLVCYTSVRWNET-
or
Sequence no. 13:
-ALETLLQNQQLLDLWGCRGRLVCYTSVRWNET-
or
Sequence no. 14:
-LNQQRLLNSWGCKGRLVCYTSV-
or
Sequence no. 15:
-RALETLLNQQRLLNSWGCKGRLVCYTSV-
or
Sequence no. 16:
-RLNSWGCKGRLVCYTSV-
or

8. Synthetic peptides according to one of claims 1 to 7, having the sequence:
Peptide no. 2 (3B)
LLSLWGCRGRLVCYTSVQWNET
or
Peptide no. 3 (4B)
LLSSWGCKGRLVCYTSVQWNET
or
Peptide no. 4 (5B)
LLSSWGCKGRLVCYTSVQWNST
or
Peptide no. 5 (6B)
LLQSWGCKGRLVCYTSVQWNST
or
Peptide no. 8 (7B)
LLSSWGCRGRLVCYTSVQWNET
or
Peptide no. 9 (12B)
LLSSWGCKGRLVCYTS
or
Peptide no. 10 (14B)
LLNSWGCKGRLVCYTS
or
Peptide no. 11 (18B)
ALETLLQNQQLLNSWGCRGRLVCYTSVRWNET
or
Peptide no. 12 (19B)
ALETLLQNQQLLNIWGCRGRLVCYTSVRWNET
or
Peptide no. 13 (20B)
ALETLLQNQQLLDLWGCRGRLVCYTSVRWNET
or
Peptide no. 14 (21B)
LNQQRLLNSWGCKGRLVCYTSV
or
Peptide no. 15 (22B)
RALETLLNQQRLLNSWGCKGRLVCYTSV
or
Peptide no. 16 (23B)
RLNSWGCKGRLVCYTSV
or

9. Composition comprising one or more synthetic peptides of the formula (I) according to any one of claims 1 to 8.

10. Composition according to claim 9 comprising peptide no. 3 (4B).

11. Composition comprising one or more synthetic peptides of the formula (I) according to claim 1 and one or more recombinant HIV-1 peptides of group O.

12. Composition comprising one or more synthetic peptides of the formula (I) according to claim 1 and one or more synthetic or recombinant HIV-1 and/or HIV-2 peptides

13. In vitro immunoassay method using one or more synthetic peptides of the formula (I) according to any one of claims 1 to 8.

14. In vitro immunoassay method using a composition according to any one of claims 9 to 12.

15. Diagnostic kit including at least one synthetic peptide of the formula (I) according to any one of claims 1 to 8 or a composition according to any one of claims 9 to 12.

## Patentansprüche

1. Synthetische Peptide des monomeren Typs mit 13 bis 33 Aminosäuren oder des dimeren Typs mit 26 bis 66 Aminosäuren in linearer Form oder in einer mithilfe von Disulfidbrücken zwischen Cysteinen cyclisierten Form der allgemeinen Formel (I):
Δ-Z-TrpGlyCys-Θ-CysTyrThrSer-Ω (I)
worin:
- Δ für einen Biotinylrest, einen Biocytinylrest, ein Wasserstoffatom, einen Acetylrest (CH₃CO-), eine aliphatische Kette, die gegebenenfalls eine oder zwei Thiol-, Aldehyd- oder Aminfunktionen trägt, steht,
- Z für eine Peptidsequenz einer der Formeln (II) bis (X) steht:
-Ξ₁-Ser-Ξ₂- (II)
-Ser-Ξ₂- (III)
-Ξ₁-Ser- (IV)
-Ξ₁-Gln-Ξ₂ (V)
-Gln-Ξ₂- (VI)
-Ξ₁-Gln- (VII)
-Ξ₁-Asn-Ξ₂- (VIII)
-Asn-Ξ₂- (IX)
Ξ₁-Asn- (X)
worin:
- Ξ₁ für eine Peptidsequenz mit 0 bis 9 Aminosäuren steht
und
- Ξ₂ für eine Peptidsequenz mit 0 bis 5 Aminosäuren steht,
- Θ für eine Peptidsequenz der Formel (XI) steht:
-(AA₁)-(AA₂)-(AA₃)-(AA₄)-(AA₅)- (XI)
worin:
• (AA₁) entweder für einen Lysinrest oder einen Argininrest oder einen Ornithinrest steht,
• (AA₂) entweder für einen Glycinrest oder einen Asparaginrest steht,
• (AA₃) entweder für einen Lysinrest oder einen Argininrest oder einen Ornithinrest steht,
• (AA₄) entweder für einen Leucinrest oder einen Isoleucinrest oder einen Glutaminrest steht,
• (AA₅) entweder für einen Isoleucinrest oder einen Valinrest oder einen Leucinrest oder einen Threoninrest oder einen Norleucinrest oder einen Norvalinrest steht,
jedoch unter der Bedingung, dass (AA₁), (AA₂), (AA₃), (AA₄) und (AA₅) niemals zusammen die Peptidsequenzen -Lys Gly Lys Leu Ile- und -Lys Gly Lys Leu Val- bilden,
- Ω, das an die Gruppe -CO- von Serin gebunden ist, Folgendes bedeutet:
- einen Hydroxylrest (-OH) oder einen Aminorest (-NH₂),
- einen Alkoxyrest mit 1 bis 6 Kohlenstoffatomen,
- eine Peptidsequenz der Formel (XII):
-Val-Σ-Ψ (XII),
worin Σ für eine Sequenz der Formel (XIII) oder der Formel (XIV) steht:
-(AA₆)-Trp Asn-(AA₇)-(AA₈) (XIII)
-(AA₆)-Trp His-(AA₇)-(AA₈) (XIV)
worin:
• (AA₆) für eine andere Aminosäure als Lysin steht,
• (AA₇) für eine Aminosäure steht,
• (AA₈) für einen Serin- oder Threoninrest steht,
und Ψ, das an den freien Rest -CO- der Aminosäure AA₈ gebunden ist, für eine Gruppe OH, NH₂ oder einen Alkoxyrest mit 1 bis 6 Kohlenstoffatomen steht,
- eine Peptidsequenz der Formel (XV):
-Val-Ψ (XV),
worin Ψ, das an den Rest -CO- von Valin gebunden ist, die gleiche Bedeutung wie in Formel (XII) hat,
- oder eine Peptidsequenz der Formel (XVI) bis (XVIII):
-Z-TrpGlyCys-Θ-CysTyrThrSer-Ψ (XVI)
Val-Σ-Z-TrpGlyCys-Θ-CysTyrThrSerVal-Σ-Ψ (XVII)
Val-Z-TrpGlyCys-Θ-CysTyrThrSerVal-Ψ (XVIII)
in denen Z und Θ der für Formel (I) angegebenen Definition gehorchen und Σ der für Formel (XII) angegebenen Definition gehorcht und Ψ, das an den Rest -CO- von Serin, an den Rest -CO- der Aminosäure AA₈ oder an den Rest -CO- von Valin gebunden ist, die gleiche Bedeutung wie in Formel (XII) hat.

2. Synthetische Peptide der Formel (I) nach Anspruch 1, worin die durch Δ dargestellte aliphatische Kette eine Alkylkette mit 1 bis 6 Kohlenstoffatomen oder eine Alkenylkette mit 2 bis 6 Kohlenstoffatomen oder eine Aminoalkylcarbonylkette mit 2 bis 6 Kohlenstoffatomen ist.

3. Synthetische Peptide der Formel (I) nach Anspruch 1 oder 2, worin (AA₅) entweder für einen Valinrest oder einen Leucinrest oder einen Threoninrest steht und, wenn Ω einer Peptidsequenz der Formel (XII) oder (XIV) entspricht, (AA₆) entweder für einen Glutaminrest oder einen Argininrest steht.

4. Synthetische Peptide der Formel (I) nach Anspruch 1 oder 2, worin
- Δ für einen Biotinylrest, ein Wasserstoffatom oder eine aliphatische Kette, die gegebenenfalls eine oder zwei Thiol-, Aldehyd- oder Aminfunktionen trägt, steht, wobei die aliphatische Kette eine Alkylkette mit 1 bis 6 Kohlenstoffatomen oder eine Aminoalkylcarbonylkette mit 2 bis 6 Kohlenstoffatomen ist,
- Z für eine Peptidsequenz einer der Formel (II) oder (V), worin Ξ₁ für eine Peptidsequenz mit zwei Aminosäuren steht und Ξ₂ für eine Aminosäure steht, oder eine Sequenz der Formel (IV), in der Ξ₁ drei Aminosäuren darstellt, oder eine Peptidsequenz der Formel (VIII), in der Ξ₁ eine Peptidsequenz mit neun, acht oder drei Aminosäuren darstellt und Ξ₂ eine Peptidsequenz mit fünf Aminosäuren darstellt, steht
- Θ für eine Peptidsequenz der Formel:
-Lys Gly Arg Leu Val-,
oder
-Arg Gly Arg Leu Val-
steht und
- Ω für eine Hydroxylgruppe, die Peptidsequenz (XV) oder eine der folgenden Sequenzen steht, die der Peptidsequenz der Formel (XII) entsprechen:
- Val Arg Trp Asn Glu Thr-Ψ,
- Val Gln Trp Asn Glu Thr-Ψ
oder
- Val Gln Trp Asn Ser Thr-Ψ.

5. Synthetische Peptide der Formel (I) nach einem der Ansprüche 1 bis 4, worin Z eine Peptidsequenz der Formel:
• -Leu Leu Ser Ser-
• -Leu Leu Ser Leu
• -Leu Leu Asn Ser-
• -Arg Leu Asn Ser-
• -Ala Leu Glu Thr Leu Leu Gln Asn Gln Gln Leu Leu Asn Ser-
• -Ala Leu Glu Thr Leu Leu Gln Asn Gln Gln Leu Leu Asp Leu-
• -Ala Leu Glu Thr Leu Leu Gln Asn Gln Gln Leu Leu Asn Ile -
• -Leu Asn Gln Gln Arg Leu Leu Asn Ser-
oder
• -Arg Ala Leu Glu Thr Leu Leu Asn Gln Gln Arg Leu Leu Asn Serdarstellt.

6. Synthetische Peptide mit 20 bis 50 Aminosäuren nach Anspruch 1 der Formel (Ia):
Δ-Zₐ-TrpGlyCys-Θ-CysTyrThrSer-Ωₐ (Ia)
worin Za für einen Rest der Formeln IIa bis Xa steht:
Ξ₁ₐ-Ser-Ξ₂ₐ (IIa)
-Ser-Ξ₂ₐ (IIIa)
-Ξ₁ₐ-Ser (IVa)
Ξ₁ₐ-Gln-Ξ₂ₐ (Va)
-Gln-Ξ₂ₐ (VIa)
Ξ₁ₐ-Gln- (VIIa)
Ξ₁ₐ-Asn-Ξ₂ₐ (VIIIa)
-Asn-Ξ₂ₐ (IXa)
-Ξ₁ₐ-Asn (Xa)
worin:
- Ξ₁ₐ für eine Peptidsequenz mit 1 bis 5 Aminosäuren steht
und
- Ξ₂ₐ für eine Aminosäure,
- Ωₐ für eine Peptidsequenz der Formel (XII), wie für Formel (I) definiert, oder eine Peptidsequenz der Formel (XVIIa):
Val-Σ-Zₐ-TrpGlyCys-Θ-CysTyrThrSerVal-Σ-Ψ (XVIIa)
steht und
Δ, Θ, Σ und Ψ die gleiche Bedeutung wie in Formel (I) haben.

7. Synthetische Peptide der Formel (I) nach einem der Ansprüche 1 bis 6, die eine der folgenden Sequenzen umfassen:
Sequenz Nr. 2
-LLSLWGCRGRLVCYTSVQWNET-
oder
Sequenz Nr. 3
-LLSSWGCKGRLVCYTSVQWNET-
oder
Sequenz Nr.4
-LLSSWGCKGRLVCYTSVQWNST-
oder
Sequenz Nr. 5
-LLQSWGCKGRLVCYTSVQWNST-
oder
Sequenz Nr. 8
-LLSSWGCRGRLVCYTSVQWNET-
oder
Sequenz Nr. 9:
-LLSSWGCKGRLVCYTS-
oder
Sequenz Nr.10 :
-LLNSWGCKGRLVCYTS-
oder
Sequenz Nr. 11 :
-ALETLLQNQQLLNSWGCRGRLVCYTSVRWNET-
oder
Sequenz Nr.12 :
-ALETLLQNQQLLNIWGCRGRLVCYTSVRWNET-
oder
Sequenz Nr.13 :
-ALETLLQNQQLLDLWGCRGRLVCYTSVRWNET-
oder
Sequenz Nr. 14 :
-LNQQRLLNSWGCKGRLVCYTSV-
oder
Sequenz Nr.15 :
-RALETLLNQQRLLNSWGCKGRLVCYTSV-
oder
Sequenz Nr.16 :
-RLNSWGCKGRLVCYTSV-
oder

8. Synthetische Peptide nach einem der Ansprüche 1 bis 7 mit der Sequenz:
PEPTID Nr.2 (3B)
LLSLWGCRGRLVCYTSVQWNET
oder
PEPTID Nr.3 (4B)
LLSSWGCKGRLVCYTSVQWNET
oder
PEPTID Nr.4 (5B)
LLSSWGCKGRLVCYTSVQWNST
oder
PEPTID Nr.5 (6B)
LLQSWGCKGRLVCYTSVQWNST
oder
PEPTID Nr.8 (7B)
LLSSWGCRGRLVCYTSVQWNET
oder
PEPTID Nr. 9 (12B)
LLSSWGCKGRLVCYTS
oder
PEPTID Nr. 10 (14B)
LLNSWGCKGRLVCYTS
oder
PEPTID Nr.11 (18B)
ALETLLQNQQLLNSWGCRGRLVCYTSVRWNET
oder
PEPTID Nr. 12 (19B)
ALETLLQNQQLLNWGCRGRLVCYTSVRWNET
oder
PEPTID Nr.13 (20B)
ALETLLQNQQLLDLWGCRGRLVCYTSVRWNET
oder
PEPTID Nr.14 (21B)
LNQQRLLNSWGCKGRLVCYTSV
oder
PEPTID Nr.15 (22B)
RALETLLNQQRLLNSWGCKGRLVCYTSV
oder
PEPTID Nr.16(23B)
RLNSWGCKGRLVCYTSV
oder

9. Zusammensetzung, die ein oder mehrere synthetische Peptide der Formel (I) nach einem der Ansprüche 1 bis 8 enthält.

10. Zusammensetzung nach Anspruch 9, die das Peptid Nr. 3 (4B) enthält.

11. Zusammensetzung, die ein oder mehrere synthetische Peptide der Formel (I) nach Anspruch 1 und ein oder mehrere rekombinante Peptide VIH-1 der Gruppe O enthält.

12. Zusammensetzung, die ein oder mehrere synthetische Peptide der Formel (I) nach Anspruch 1 und ein oder mehrere synthetische oder rekombinante VIH-1- und/oder VIH-2-Peptide enthält.

13. Verfahren zur immunologischen In-vitro-Quantifizierung unter Verwendung eines oder mehrerer synthetischer Peptide der Formel (I) nach einem der Ansprüche 1 bis 8.

14. Verfahren zur immunologischen In-vitro-Quantifizierung unter Verwendung einer Zusammensetzung nach einem der Ansprüche 9 bis 12.

15. Diagnostisches Kit, das ein oder mehrere synthetische Peptide der Formel (I) nach einem der Ansprüche 1 bis 8 oder eine Zusammensetzung nach einem der Ansprüche 9 bis 12 enthält.
